# EUROPEAN PATENT SPECIFICATION

(11) **EP 1 779 119 B1**
(45) Date of publication and mention of the grant of the patent: **01.08.2012**
(21) Application number: 05788460.3
(22) Date of filing: 17.08.2005
(51) Int. Cl.: G01N 33/92, C12Q 1/60

(54) **NON-PRECIPITATING BODILY FLUID ANALYSIS METHOD**
NICHTKOMPLEXBILDENDES KÖRPERFLÜSSIGKEITSANALYSEVERFAHREN
PROCEDE D'ANALYSE D'UN FLUIDE CORPOREL NE PRECIPITANT PAS

(30) Priority: 17.08.2004 US 602210 P; 11.10.2004 US 962272
(43) Date of publication of application: 02.05.2007
(73) Proprietor: Polymer Technology Systems, Inc., Indianapolis, IN 46268 (US)
(72) Inventor: LAWRENCE, Gregory, M., Indianapolis, IN 46220 (US); KNIGHT, Meredith, Indianapolis, IN 46214 (US)
(74) Representative: Rottmann, Maximilian
(86) International application number: PCT/US2005/029458
(87) International publication number: WO 2006/023678

(56) References cited:
- EP-A- 0 597 268
- EP-A- 0 753 583
- WO-A-00/73797
- DE-A1- 3 217 925
- US-A- 5 135 716
- US-A1- 2003 166 291
- US-A1- 2004 126 830
- US-A1- 2005 170 447
- US-B1- 6 171 849
- G.R. WARNICK, M. NAUCK, N. RIFAI: "Evolution methods for measurement of HDL-cholesterol: from ultracentrifugation to homogeneous assays" CLINICAL CHEMISTRY, vol. 47, no. 9, 2001, pages 1579-1596, XP002357904
- M. NAUCK, G.R. WARNICK, N. RIFAI: "Methods ofr measurement of LDL-cholesterol: a critical assessment of direct measurement by homogeneous assays versus calculation" CLINICAL CHEMISTRY, vol. 48, no. 2, 2002, pages 236-254, XP002357905
- SUGIUCHI H ET AL: "Homogeneous assay for measuring low-density lipoprotein cholesterol in serum with triblock copolymer and alpha-cyclodextrin sulfate", CLINICAL CHEMISTRY, AMERICAN ASSOCIATION FOR CLINICAL CHEMISTRY, WASHINGTON, DC, vol. 44, no. 3, 1 March 1998 (1998-03-01), pages 522-531, XP002382301, ISSN: 0009-9147

## Description

### BACKGROUND OF THE INVENTION

### 1. Field of the Invention

The invention in general relates to bodily fluid analysis systems including a disposable test strip and a spectrophotometric sensing device, with particular application to on-site testing of particular analytes in blood.

### 2. Statement of the Problem

The level of certain analytes in blood and other body fluids is often used to diagnose disease, determine disease risk factors, monitor the course of a therapy, or determine the presence of illicit drugs. For example, analytes carried in blood have been evaluated to determine various cholesterol and triglyceride levels as a significant indicator of risk of coronary heart disease.

The blood analysis necessary to determine bodily fluid analytes, such as total cholesterol, high density lipoprotein cholesterol (HDL), low density lipoprotein cholesterol (LDL), and triglycerides, may be performed in clinical setting in a laboratory or on site using dry test strips. In the laboratory, the blood is centrifuged to separate the red blood cells from the plasma, and carefully controlled chemical tests in test tubes are performed to determine the concentration of analytes. Dry test strips utilize several membrane layers to separate red blood cells from blood plasma, react the plasma with a particular reagent or reagents, and obtain a signal indicative of the concentration of a particular analyte, which is usually a spectrophotometric signal. See, for example, United States Patent No. 4,774,192 issued September 27, 1988 to Terminiello et al.; United States Patent No. 4,477,575 issued October 16, 1984 to Peter Vogel et al.; United States Patent No. 5,104,619 entitled "Disposable Diagnostic System"; United States Patent No. 5,135,716 issued August 4, 1992 to Tatin B. Thakore; United States Patent No. 5,166,051 entitled "Membranes, Membrane Overlays, For Exclusion of Erythrocytes, And Method Of Immunoassay of Whole Blood Analytes"; United States Patent No. 5,597,532 issued January 28, 1997 to James Connolly; United States Patent No. 6,171,849 issued January 9, 2001 to Walter Rittersdorf et al.; United States Patent No. 6,759,190 issued July 6. 2004 to Jinn-Nan Lin et al., United States Patent Application Publication No. US2004/0126830 published July 1, 2004 on an invention of Bruce Shull et al.; and United States Patent Application Publication No. US2005/0003523 published January 6, 2005 on an invention of Sunil Anaokar et at al.

Sugiuchie et al. ("Homogenous assay for measuring low-density lipoprotein cholesterol in serum with triblock copolymer and α-cyclodextrin sulphate" In Clinical Chemistry, Vol. 44, No. 3, 552-531 discloses an assay for measuring low-density lipoprotein cholesterol in serum with triblock copolymer (POE-POP) and α-cylcodextrin sulphate. The use of POE-POP reduces the reactivity of cholesterol, especially in HDL, suggesting that a combination of POE-POP with α-cylcodextrin sulphate could provide the needed selectivity for the determination of LDL-C in serum. The human sera were isolated by ultracentrifugation according to known methods. The lipoprotein fractions were separated by gel filtration analysis. For the determination of total cholesterol in serum, excess amounts of surfactants have been used for solubilizing all the lipoprotein cholesterol in a non-specific manner and allowing it to freely participate in the enzymatic reaction system. For the direct measurement of LDL-C in serum the sequential two surfactants are used that show differential selectivities towards lipoprotein fraction.

All of the above systems depend on precipitation and/or flltration to separate the unwanted components from the analytes to be tested. For example, if HDL Is the desired analyte, the other lipoproteins are reacted to for a precipitate and are filtered from the plasma using filter membranes. However, the precipitates tend to block the pores in the system and Impede the flow of the desired analyses also, which reduces the amount of the desired analytes that reach the reaction area, and thus reduces the accuracy of the test. The conflict between the need for good separation of unwanted components from the analytes and the accuracy problems associated with such separation has caused the accuracy of the test strip/spectrophotometric systems to plateau, and has limited the usefulness of this art. Thus, there is a need for a test sttip/spectrophotometer architecture that can improve the capabilities of the dry strip technology system and that yield more accurate readings.

### SUMMARY OF THE INVENTION

The present invention solves the above problem by providing a dry test strip chemistry that reacts the unwanted components of the bodily fluid into complexes that do not participate in the test reaction. The complexes remain free to flow, and thus do not clog membranes or filters.

The invention further comprises a method of determining a characteristic of a selected one of a plurality of analytes in a bodily fluid, according to claim 1.

These and other objects and benefits of the invention will become apparent from the following-written description and accompanying figures.

### BRIEF DESCRIPTION OF THE DRAWINGS

FIG. 1 is an exploded perspective view of the preferred embodiment of a test strip assembly according to the invention;
FIG. 2 is a cross-sectional plan view of test strip of FIG. 2 taken through the line 2 - 2 of FIG. 1;
FIG. 3 is a bottom plan view of the cap portion of the test strip assembly of FIG. 1;
FIG. 4 is cross-sectional view of the test strip holder of FIG. 1;
FIG. 5 is a cross-sectional view of a test strip element according to the Invention illustrating some of the features of the invention;
FIG. 6 is a graph showing the data-derived baseline reflectance versus mg/dl HDL curve from which a reflectance test according to the invention can be generated;
FIG. 7 is a graph of HDL cholesterol readings from a dry test strip according to the invention plotted along the ordinate versus reference HDL cholesterol for the same sample plotted along the abscissa; and
Fig. 8 Illustrates another preferred embodiment of a test assembly according to the invention In which there are a plurality of strip holders and test strips.

### DETAILED DESCRIMON OF THE PREFERRED EMBODIMENT

For the purposes of promoting an understanding of the principles of the Intention, reference will now be made to the embodiments illustrated in the drawings and described in the following written specification. drawings and relative size of the various parts are generally altered so as to better illustrate the invention within the constraints of a written document such as this.

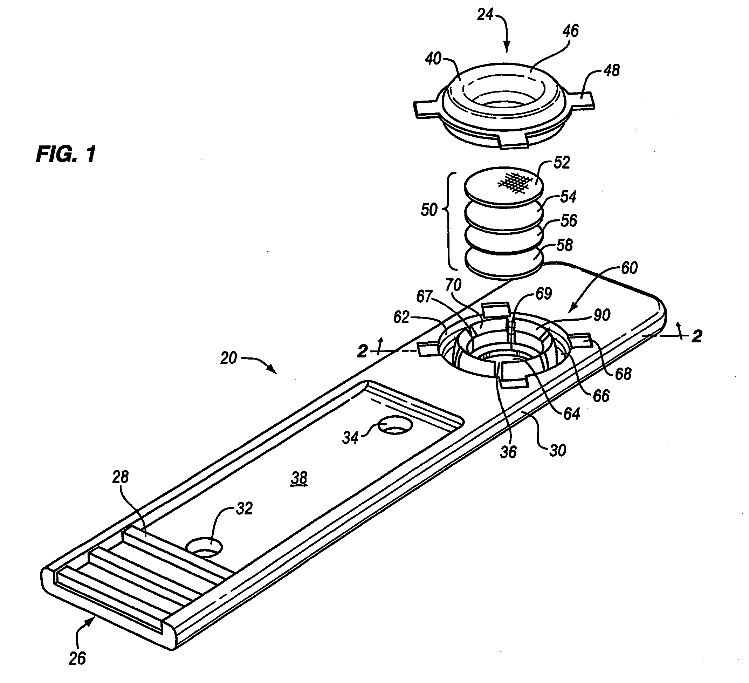

An exploded perspective view of an exemplary test assembly 20 according to the invention is shown in FIG. 1. Test assembly 20 includes a preferably elongated test strip carrier body 30, a test strip 50, and a test strip holder 24. Test strip holder 24 includes a holder base portion 60 and a holder cap 40. Carrier body 30 include a grip portion 26, openings 32 and 34, sensor port or test opening 36, and holder base 60. Grip portion 26 Includes raised ribs 28 which permit the fingers to easily grip the carrier body 30.

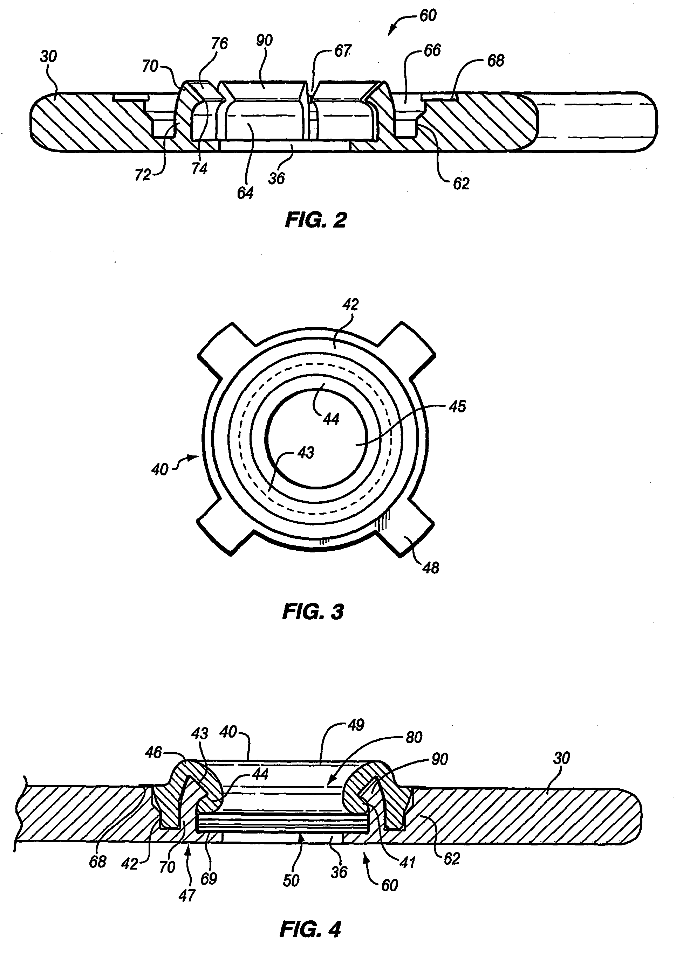

The holder base 60 is shown in FIGS. 1, 2, and 4. FIG. 1 shows a perspective view, FIG. 2 shows a partially cross-sectioned side view, and FIG. 4 shows a cross-sectional view with the cap 40 In place over holder base 60. Preferably, holder base 60 includes a well 62 formed in body 30, alignment recesses 68, and retainer 90, which is preferably flexible. Well 62 has an upward sloping well wall completely encircling the test opening (sensor port) 36. Retainer 90 preferably comprises fingers 70 and separates well 62 into an inner portion 64 which forms a test strip well 64 and an outer portion 66, which is preferably relatively small in volume, being just big enough to allow fingers 70 to flex. In this disclosure, the term "encircle" does not necessarily mean the encircling structure forms a circle, but rather It has the broader common meaning of "to pass completely around". In the preferred embodiment, however, the wen 62 and fingers 70 do form a circle. In the preferred embodiment, there are four alignment recesses 68 and six fingers 70, though the invention contemplates that any number suitable to perform the functions described below may be used. Each finger 70 includes a stern portion 72, a hook portion 74, and a ramp portion 76 that is preferably formed at an acute angle to a vertical line perpendicular to the plan of body 30. Fingers 70 are separated by channels 67. The bottom of well 62 forms a test strip support 69 around port 36 on which, as will be seen below, the test strip 50 rests, as best shown in FIG. 4.

Cap 40 is shown in FIGS. 1, 3, and 4. FIG. 1 shows a perspective view, FIG. 3 shows a bottom plan view, and FIG. 4 shows a cross-sectional view of the cap 40 in place over the holder base 60. Cap 40 includes an outer foot 42, an inner flange 44, and a connecting portion 46, which, as will be seen below, forms the brim 49 of a bodily fluid container 80. The outer foot 42 and inner flange 44 have different lengths, with the inner flange being shorter. The difference in lengths is less than the thickness of test strip assembly 50. so that the inner flange 44 and test strip support 69 engage strip 50 sufficiently to secure it in place. Preferably, the difference is sufficient so that flange 44 and test strip support 69 compress strip 50 between them. The bottom 43 of connecting portion 46 is shaped to form a groove 47 into which fingers 70 fit snuggly. A lip 41 is formed on flange 44 (FIG. 4 which engages hook 72 to latch cap 40 on holder base 60. The distal end 84 of flange 44 is smooth and rounded so as not to damage test strip 50.

Test strip 50 is shown in FIGS. 1 and 4, and is preferably formed of a plurality of layers. Each layer pertorms a specific function as required by each specific test. Generally, there Is a "spreading" layer 62 to ensure even distribution of the whole blood sample; a "separation" layer 56 to obtain a clarified plasma/serum sample; a layer or layers 54 to hold specific test reagents In sequence as needed by each specific assay, and a final "color" or "test reaction" layer 59 to provide a matrix on which a specific color or test reaction will develop for each specific test The order of the layers can vary. For example, the separation layer may come before or after the reagent layer(s). The details of the test strip layers is described below.

The test strip assembly 20 is assembled as shown in FIGS. 1 and 4. A cone-shaped inserter (not shown) presses down on the ramps 76 of the fingers 70 and spreads them sufficiently to drop the assembled test strip 50 onto test strip support 69. Cap 40 is then pressed home on retainer 90, with fingers 70 forced into groove 47, compressing test strip 50 sufficiently to hold it In place.

The carrier body 30, holder base 60, and cap or cover 40 are preferably made of plastic or other suitable material. The preferred plastics are polypropylene or nylon, though other plastics may be used. Preferably, the plastic parts are injection molded, and cap 40 is sonic welded to holder base 60 at locator tabs 68. Thus, the placement tabs enable the cap to be welded without contact with the main body of cap 60. Preferably, the plastic parts, particularly the cap 40, are color-coded to correspond to the particular test, such as HDL, LDL, total cholesterol, etc., for which the test strip assembly, such as 50, is designed.

Preferably, for the exemplary HDL test, there are four layers 52, 54, 56, and 58, best shown in FIG. 1. Top layer 52 is preferably a spreading layer designed to disperse the bodily fluid rapidly In all horizontal directions so that it is distributed evenly across the test strip 50. Another function of layer 52 is to distribute the pressure exerted by the cap or cover 40, as evenly as possible acrose the entire area of the lower layers, such as 54, 56, and 58. Thus, it should be fairily stiff. Preferably, it should be sufficiently stiff to provide a flat surface; that is, a surface with a bulge in the middle of less than 0,0508 mm (.002 Inches) when the cap is in place, but sufficiently flexible to allow the cover to seal the edge of the membranes. Preferably, layer 52 is made of a mesh with either an open or closed weave. Some suitable woven mesh materials are SEFAR^{™} type 78 SK 022, which is an open mesh with a close weave, or a Tetko^{™} mesh, which is a closed mesh, though other suitable and equivalent materials may also be used. An open mesh works by letting the sample through, while a closed mesh works by adhesion of the sample to the mesh threads, i.e., by wicking. Thus, different parameters are required for the different meshes, If an open mesh is used, preferably more than 40% of the total area should be open, and more preferably 50%. If the mesh is a closed mesh, the open area should be 15% or less and more preferably 10% or less.

The next layer 54 contains the reagents that interact with the non-desired analytes that would compromise the colorimetric test to be performed in layer 58 so that these analytes do not participate in the colorimetric reaction. For example, if the colorimetric test in layer 58 is to be a test for HDL, analytes, such as LDL (low density lipoproteins), VLDL (very low density lipoproteins), ILDL (intermediate density lipoproteins), and chylomicrons (big, tryglyceride-rich lipoproteins) that may make the test less accurate or reliable are interacted with in some way that prevents them form participating in the colorimetric reaction in layer 58. Preferably, the reaction is one in which these analytes are bound In clusters within a compound that prevents them from reacting. An example of the specific reagents are given in Example 1, below.

Layer 54 is also preferably a depth filter, which functions to reconstitute the reagent; that is, get the dried reagent into solution. A key feature of this layer 54 is that it includes many small fibers, and thus it has a large surface area. Preferably, the fibers are random, that is, they are not organized as in a weave. This type of filter is often referred to as a conjugate relief pad, wicking pad, sample pad, or prefilter. The surface area is preferably such that the wicking rate is below 8 seconds per two centimeters. Preferably, the surface area should be such that after wetting with the reagent and drying, the layer holds a weight of dry reagent equal to the membrane weight itself. Preferably, the weight of the dry agent should not be lower than 75% of the weight of the membrane and not above 125% of the weight of the membrane. Since the reagent is on the surface of the fibers, the large surface area helps to reconstitute the reagent faster, since there is a larger area of reagent exposed to the solvent. Preferably, the average pore size of this layer is controlled to optimally control flow through the layer so that the bodily fluid remains long enough to reconstitute the reagent, but not so long as to delay or otherwise hinder the test in layer 58. The controlled pore size in combination with the large surface area helps to limit or retard the movement of the solute in the vertical direction, so that it remains in the material longer, and thus has more time to dissolve the reagent. Preferably, layer 54 is made of a non-woven, fibrous material such as a hydroxylated polyester, preferably a polyhydroxylated polyester. Suitable such materials are membranes made by Pall Life Sciences, such as Accuwik Ultra™. Preferably, the membrane is inserted with the bumps side down.

The purpose of the next layer 56 is preferably to remove red blood cells from the analyte liquid and to further add to the reagent/solvent contact lime to continue the process of getting the reagent into solution. It is preferably made of an asymmetrically porous material; that is, the pore size varies through the material. Preferably, the side with the large pores is up. In the preferred embodiment, it has a pore size of between 250 µm (microns) and 350 µm (microns), and more preferably 300 µm (microns) on the sample-receiving side, and a pore size of between 0.5 µm (microns) and 10 µm (microns), and preferably 3 µm (microns), on the detection side. The preferred material is an asymmetric polysulfone such as is BTS-SP-300 or BTS-SP-200 available from Pall Life Sciences, or other suitable materials may be used. Other suitable materials are lechtin-coated graphite fibers, ruthenium oxide fiber, and other materials known in the art. The asymmetric nature of the layer 56 is affective in removing red blood cells while continuing the movement of the solvent and reactant downwards. In the preferred embodiment, it removes the red blood cells by slowing them as they percolate through the tortuous path of the pores. As the pores get smaller, the red blood cells may also become entangled In the fibers, but this happens gradually and relatively randomly throughout the layer, rather than collecting all at one level within the test strip, as they would in a conventional filter with a single pore size; such collecting all at one level tends to block fluid flow. The relatively random entrapment of the red blood cells leaves open capillary paths through the material. Such capillaries assist in drawing the fluid downward through the test strip 50, particularly since the capillaries become smaller in that direction. As will be seen more clearly below, however, it is only necessary to slow the red blood cells to separate them. That is, because the bottom of container 80 is essentially closed, flow stops when the layer 58 becomes saturated. If flow stops and the red blood cells are still in the upper layers, they will remain there.

Bottom layer 58 is the detection layer and contains the detection reagent It is preferably made of a hydrophobic material which has sufficient surface tension with the analyte bodily fluid so that the fluid will not flow past it. In the preferred embodiment, the test strip assembly layers 52 - 58 are circular and are all of the same diameter, though other shapes and sizes may be used. The preferred detection layer 58 is the Biodyne^{™} A membrane available from Pall Corporation with the total cholesterol formulation described in United States Patent Application Publication US 2004/0126830 on application Serial No. 10/663,555 filed September 16, 2003. This membrane is a nylon membrane in which the net charge can be controlled by changing the pH. As disclosed In the forgoing reference, the reagents are Trinder reagents which include enzymes, such as cholesterol oxidase, perosidase, and cholesterol esterase, that react with cholesterol to effect a color change which can be detected optimally.

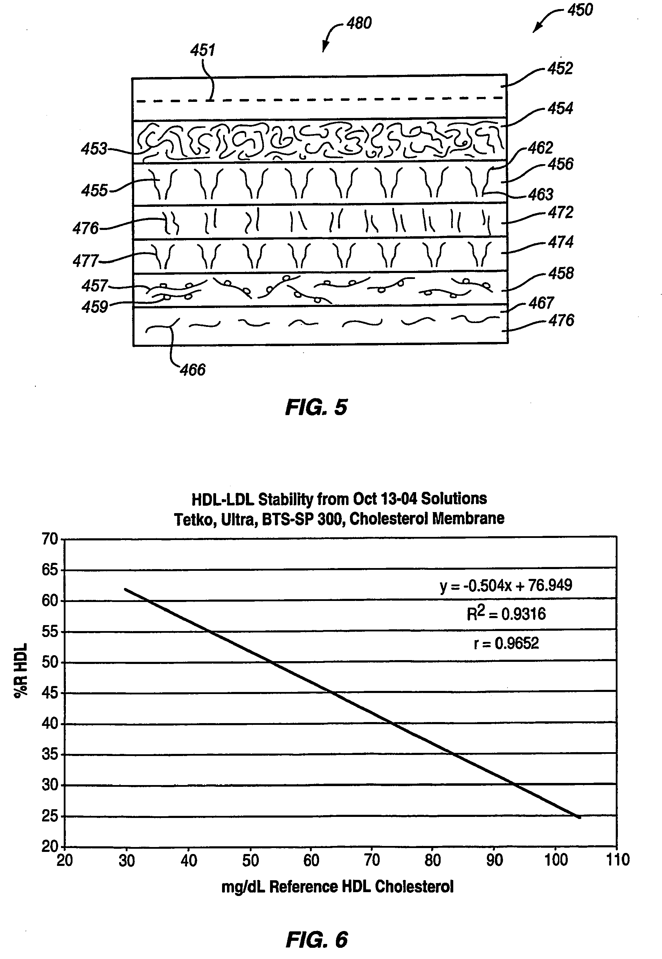

FIG. 5 is a cross-sectional view of an alternative test strip assembly 450 according to the invention illustrating some of the features of the invention. Test strip assembly 450 includes layers 452, 454, 458, 472, 474, 458, and 476. Layer 452 comprises a woven mesh 451. Weaves tend to cause fluid to flow more easily along the weave rather than through it, and thus, if the weave 451 is horizontal, layer 454 will tend to distribute the bodily fluid across the layer. Layer 454 can either be a material that traps and holds red blood cells, such as Tuffglass^{™}, or it can be a material such as Accuwick Ultra^{™}, that merely slows the red blood calls. Preferably, it includes fibers 453 that are relatively randomly distributed. That is, the fibers 453 are not organized as in a weave. Preferably, the fibers are also very thin, and thus the layer 454 has a large surface area. This type of material holds a relatively large amount of fluid, and the fluid is in contact with a lot of area. This material functions well to get reagents on the surface of the fibers into solution. Layer 456 is a membrane material. Membranes have pores that are relatively organized. The preferred material of layer 458 is an asymmetric membrane, which means that the pores vary in size. Preferably, in layer 456 the pores are larger at the upper end 482 of the material and smaller at the lower end of the material 463. Note that for illustration purposes the pores are shown in layer 456 as single channels with a varying diameter, but in fact the "channels" are preferably not well-defined and branch in all directions. The important characteristic is that the dimensions of the pores are larger at end 462 than at the other end 483. In the preferred embodiment, the membrane used is more like a depth filter at the top; that is, the material is fiber-like and amorphous. That is, the fibers are disorganized, i.e., essentially randomly distributed. At the bottom it is membranous, with a definite pore size. The layer can be engineered to be more or tests depth filter-like at the top and more or less like and absolute membrane at the bottom. The more it is like a depth filter, the more capacity it has. The preferred material is a polysulfone.

Layers 472 and 474 are preferably optional layers used in controlling timing of the reconstitution of the reagent in layer 456. For example, membrane 472 may be a Supor^{™} 1200 untreated membrane. This example has relatively large 1200 µm (micron) pores. It is used to slow down the percolation of the analyte liquid through the assembly to give the reagent introduced in layer 456 more time to dissolve. The smaller the pores in layer 472, the more it slows down the analyte. Layer 474 is an optional layer, preferably having asymmetrical pores 477, that may be identical to layer 456, and is included if it is desired to put more reagent in solution, or to put less reagent in layer 456 so that it dissolves more easily. Layer 458 is a reagent layer which is illustrated by showing a fiber 467 with a reagent 459 on its surface, This reagent is the colorimetric reagent that reacts with the analyte to produce the color, the reflectance of which provides the test result. Layer 476 is a layer in which the individual fibers 466 are preferably hydrophobic, which means they tend to repel water; that is, preferably, water has a high surface tension on the material. Water will tend not to penetrate this material. However gas, such as air, will pass easily through this material. Preferably, the material of layer 476 is an open pore material, and/or also holds a relatively large amount of fluid, as compared to membranes such as 456. However, it also may be an asymmetric membrane with the larger pores on the upper side 467. Such a material tends not to hold large amounts of fluid, but makes the fluid available to the reaction layer 458, as will be discussed in more detail below. Layer 476 is also preferably very thin and/or transparent, particularly when it is saturated with liquid, so that the color in layer 458 can be sensed through it.

The test strip operates generally as follows. A drop of bodily fluid, such as blood, is placed within the sample application port 45 of cap 40. It is evenly dispersed across the opening by test strip layer 52 and percolates vertically downward. The pall membrane 54 separates the unwanted material, such as the red blood cells, from the rest of the fluid, such as the serum. The red blood cell filtration/reagent membrane 56 includes reagents that react with undesired analytes that would compromise the test in membrane 58. For example, if the test in membrane 58 is for HDL, the LDL, ILDL, VLDL, and chylomicron portions of the serum are complexed in membrane 56. The membrane tends to slow or retain the complexed lipoproteins, but allows the HDL to pass to reagent layer 58. The HDL reacts in reagent layer 58 to turn the layer a predetermined color, which is detected by spectrophotometer device 10. However, it is not necessary that membrane 56 retains or even slows the complexed undesired lipoproteins. The complexing itself prevents the undesired analytes from participating in the reaction in test membrane 58 and thus takes these analytes out of the reaction that determines the color. A more detailed description of the chemicals used in the test strip layers and the chemical reactions that take place in the test strip layers will be presented below.

The chemistry of the test strip element 50, 450 is selective of specific lipoproteins by being able to keep them from reacting in layer 58, 458 and/or enhancing their reaction in layer 58, 458 depending on differences between the size, mass density, and surface charge density of the HDL, LDL, ILDL, VLDL, and chylomicron lipoproteins. As known in the art, the HDL lipoproteins are the smallest, have the greatest mass density, and the highest surface charge density; the VLDL and chylomicrons are the largest, have the smallest mass density, and the lowest surface charge density; and the others are in between. It is sometimes helpful to think of HDL as a baseball, the LDL as a small beach ball, and the VLDL as a very large beach ball. The chemistry for an HDL test strip relies on a complex including a polyanion, a divalent metal, and the lipoproteins. Preferably, the polyanion is a negatively charged polymer. Preferably, the polymer is dextran sulphate. The divalent metal forms a salt bridge between the lipoprotein and creates a polymer complex that shields the cholesterol from surfactant emulsification required for it to participate in the Trinder enzymic reactions that create the color change in the layer 58, 458. For this complex to be selective between the various lipoproteins, the molecular weight, charge density, and branching of the anionic polymer must all be considered. To be selective without precipitation, the molecular weight should preferably be between 50,000 and 8,000; more preferably between 25,000 and 10,000; and most preferably between 18,000 and 12,000. The charge density should roughly match the lipoproteins you are trying to bind, with the proviso that the more branching there is, the less the charge that is required. With these parameters adjusted for LDL, ILDL, VLDL molecules, and chylomicrons, the complex does not form well with the HDL, because the polymer molecules are too large and possess too small a surface charge density to bind easily with the small, dense HDL molecule. However, the polymer molecules bind easily with the LDL, ILDL, VLDL molecules, and chylomicrons, complex them, and take them out of the reaction. Thus, the reaction occurs essentially only with the HDL molecules, and results in an effective HDL assay.

The chemistry for an LDL assay is similar, but somewhat more complicated due to the fact that the LDL is intermediate between the HDL and ILDL, VLDL molecules. This chemistry is disclosed in detail in copending and co-owned United States Patent Application Serial No. 10/962,272 filed October 11, 2004 on an invention of Greg Lawrence and John Pasque. In this chemistry, the anionic polymer is the same, but a surfactant is selected that is specific to the LDL. That is, a destabilizing agent specific to the LDL is added that enables the LDL to react more quickly with the Trinder enzymes. As disclosed in the above-referenced application, this destabilizing agent may be a glycol, such as polypropylene glycol or polyethylene glycol, and is preferably a polyoxyethylene-polyoxypropylene-polyoxyethylene hybrid, and more preferably such a hybrid having a molecular weight between 2,100 and 6,000, and most preferably with a preponderance of polyoxyethylene. Such destabilizers act by loosening the bonds just beneath the surface of the lipoprotein, penetrating the surface, and expanding it to permit the entry of surfactants which solubilize the cholesterol and make it available for the Trinder reactions. Because of the high density surface of the HDL, they do not penetrate it easily. In fact, the compounds tend to complex with the surface of the HDL molecule and isolate it from the Trinder reactants. The compounds are able to penetrate the ILDL, VLDL, and chylomicron molecules, but because these molecules are so large, the effect is diminished.

The choice of surfactant is based on a number of factors. As indicated, the properties of the surfactant are such that the complexes to be measured are selectively emulsified. Further, the destabilizing compounds, such as polypropylene glycol, are not very water soluble. Thus, to get them to act on the lipoproteins, they are preferably emulsified by a surfactant. If the surfactant is too strong in the LDL assay, non-LDL lipoprotein cholesterols are emulsified and subsequently react, and the process is non-selective. Thus, a gentler surfactant, such as CHAPS (3-{[3-Cholamidopropyl]dimethylammonio}3-propane-sulfonate) or other pluronic non-ionic surfactants should be used. A stronger surfactant, such as Triton X-100, can be used in the HDL assay because the ILDL, VLDL, and chylomicron molecules are not destabilized.

### EXAMPLE I

**Table A**

| Accuwick Ultra | | | |
|---|---|---|---|
| Solution ID SolOct13-04 A4 | | | |
| Item Description | Lot Number/Batch | Total Mass g | % Used |
| Lab D.I Water | | 177.34 | 88.6688 |
| Dexhalip 50 | Warnick & Co. 00501 Lot 99123 | 150 | 0.7500 |
| Mops Buffer | | 1.16 | .5813 |
| Sorbitol | Sigma-Item S-7547 Lot 70K0936 | 20.00 | 9.9999 |
| Ph Adjustment | 5N NaOH/HCl | Adjust pH to target 7.20 | |
| Total | | 200.00 | |
| Cloud Point | 1% MOPS = 48mM | Used 48mM TRIS | |

**Table B**

| Accuwick Ultra | | | |
|---|---|---|---|
| Solution ID SolOct13-04 A4 | | | |
| Item Description | Lot Number/Batch | Total Mass g | % Used |
| SolOct13-04 A1 (Table A) | | 29.54 | 98.4667 |
| MgCl2*6H20 | Sigma: Item M-9272; Lot 70K09321 | 0.46 | 15333 |
| pH Adjustment | 5.0 N NaOH/HCl | Adjust pH to target 7.20 | |
| Total | | 30.00 | |
| Cloud Point | 1% MOPS - 48mM | Used 48mM IRIS | |

**Table C**

| BTS Formulation | | | |
|---|---|---|---|
| Solution ID SolMar28-05 C | | | |
| Item Description | Lot Number/Batch | Total Mass g | % Used |
| Lab D.I. Water | | 276.00 | 92.0000 |
| Mops Buffer | Sigma M-1254 Lot 71K5450 | 3.00 | 1.0000 |
| Sorbitol | Sigma-Item S-7547 Lot 70K0936 | 9.00 | 3.0000 |
| Sucrose | Sigma-Item S-5016 | 9.00 | 3.0000 |
| MgCl2*6H20 | Sigma: Item M-9272; Lot 70K09321 | 3.00 | 1.0000 |
| PVA 30-70**K** | | 8.00 | 2.667 |
| pH Adjustment | 5N NaOH/HCl | Adjust pH target 7.20 | |
| Total | | 300.00 | |
| Cloud Point | | | |

A sheet of material 54 was made in the following manner. A dextran sulphate sodium salt In the form of Dextralip^{™} 50, which has a molecular weight of about 40,000, MOPS buffer (3-marpholinopropanesulfonic acid) and sorbitol were added to deionized (DI) water in the amounts shown in Table A, mixed, and adjusted for pH as indicated in the table to make a stock solution. As known in the art, pH is adjusted as necessary to the alkaline side with NaOH, and to the acid side using NCL. Then to 29.54 grams of the stock solution, MgCl₂·6H₂0 was added in the amount shown in Table B, and the pH was again adjusted. The Accuwick Ultra^{™} material was dipped into this solution and was hung vertically to allow the excess solution to drip off the material. The material was then dried horizontally in drying tunnel at 29.4 - 35.0 °C for 9 minutes to 18 minutes.

A sheet of material 56 was made in the following manner. Mops buffer, sorbitol, sucrose, MgCl₂·6H₂0, and polyvinyl alcohol 30-70K were added to DI water in the amounts shown in Table C. A sheet of BTS material was dipped into this solution and was hung vertically to allow the excess solution to drip off the material. The material was then dried horizontally in a drying tunnel at between 21.1 - 32.2 °C and more preferably between 23.8 - 29.4 °C for 9 minutes to 18 minutes.

A sheet of material 58 was made as follows. A stock solution of Cholesterol Foundation was made with 800 g DI water, 30 g sodium citrate (dihydrate), 60 g of polyvinyl propylene K-30, 2 g benzoic acid, 4 g BSA, and 1.47 g EDTA (disodium, dihydrate). The pH was adjusted to about 5.5, and then sufficient DI water was added to make 1000 ml of solution. Then a solution was made with 200g DI water, 0.771 g Triton X-100, 532 g cholesterol foundation, 13.88 g BSA, 95.61 g 10%
Gantrez AN-139 (w/v), 19.82 g CHAPS (3-{[3-Cholamidopropyl]dimethylammonio} 3-propane-sulfonate), and 37.01 g sucrose, and the solution was adjusted to a pH of about 5. Then 0.116 g of potassium ferrocyanide, 0.37 g TOOS, 4.63 g MaOS, 148 KU cholesterol oxidase, 462.6 KU perosidase, 92.5 KU cholesterol, esterase, and 4.163 g 4-Amino antipyrine, and the pH of the solution was adjusted to about 5.4. Enough DI water was added to make 1000 ml of solution. A sheet of Biodyne^{™} A material was dipped into this solution and was hung vertically to allow the excess solution to drip off the material. The material was then dried horizontally in a drying tunnel at between 32.2 and 37.7 °C for 9 minutes to 18 minutes.

A test strip assembly 50 was made by assembling a sheet of SEFAR^{™} type 76 SK 022 and the above three sheets. Circular blanks were cut out and were inserted in a test assembly 20 as shown in FIGS. 1 and 4. In a manner known in the art, a curve as shown in FIG. 6 was then constructed using reflectance measurements from a standard laboratory test for HDL. As also known in the art, the curve of FIG. 6 was then used to program a Bioscanner 2000 reader available from Polymer Technology Systems, Inc., Indianapolis, IN. The reader was then successfully used to directly read HDL concentrations In milligrams per deciliter (mg/dl) from a test assembly as described above.

### EXAMPLE II

**Table D**

| Accuwick Ultra or other Depth Filter Treatment | | | |
|---|---|---|---|
| Solution Fractionation: Solution D | | | |
| Item Description | Lot Number/Batch | Total Mass g | % Used |
| Lab D.I Water | | 175.0 | 87.5 |
| Dextralip 15 | Warnick & Co. | 0.80 | 0.4008 |
| Tris Buffer | | .0.62 | 0.31 |
| Sorbitol | Item S-7547 Lot 70K0936 5N NaOH/3.25N HCl | 10.7 | 5.34 |
| Ph Adjustment | | Adjust pH to target 7.20 | |
| Total QS to Final Weight | | 200.00 | |
| Cloud Point | | | |

**Table E**

| Asymmetric Membrane (BTS-SP-300) | | | |
|---|---|---|---|
| Solution Selectively:Solution E | | | |
| Item Description | Lot NumberBatch | Total Mass g | % Used |
| Lab D.I Water | | 175.0 | 87.5 |
| PVA30-70K | | 2.0 | 1.0 |
| Mops Buffer | Sigma:Item M-1254; Lot 5450 | 1.0 | 1.5 |
| Sorbitol | Sigma-Item S-7547 Lot 70K0936 | 3.0 | 1.5 |
| Sucrose | Sigma-Item S-5016 | 3.0 | 1.5 |
| MgCl2*6H2O | Sigmaz:Item M-9272; Lot 70K09321 | 1.0 | .5 |
| pH Adjustment | 5.0 N NaOH/HCl | Adjust pH to target 6.40 | |
| Total | | 30.00 | |
| Cloud Point | 1% MOPS-48mM | | |

A sheet of material 54 was made in the following manner based on the solution composition identified in Table D. A dextran sulphate sodium salt In the form of Dextralip^{™} 15, which has a molecular weight of about 12,000, TRIS buffer (TRIS Hydroxymethyl Aminomethane) and sorbitol were added to deionized (DI) water in the amounts shown in Table D, then mixed and adjusted for pH as indicated In the table to make the impregnation solution. As known in the art, pH was adjusted as necessary to the acidic side with 3.26 N HCl. The final impregnation solution was QS'ed (adjusted with Quantity Sufficient) with D.I. water, to the final target weight as set forth in Table D. The pH was again tested and adjusted as required by the methods previously disclosed. The Accuwick Ultra^{™} material was dipped into this solution and was hung vertically to allow the excess solution to drip off the material. Alternatively, larger membrane treatments are accomplished by the use of a drying tunnel In either the vertical, horizontal, or inclined arrangement. In practice, the material was then dried in an inclined position in a drying tunnel at between 21.1 and 37.7°C and more preferably between 26.6 and 32.2°C for 9 minutes to 18 minutes.

A sheet of material 56 was made In the following manner. Mops buffer, sorbitol, sucrose, MgCl₂·OH₂O and polyvinyl alcohol 30-10K were added to DI water in the amounts shown in Table E. A sheet of BTS, an asymmetric polysulfone membrane, was dipped into this solution and was hung vertically to allow the excess solution to drip off the material and allowed to dry at room temperature. Alternatively, larger membrane treatments are accomplished by the use of a drying tunnel in either the vertical, horizontal, or inclined arrangement. Preferably, the material is dried in an inclined position in a drying tunnel at between 21,1°C and 32,2°C (70°F and 90°F), and more preferably between 23,3°C and 29,4°C (75° and 85°F), for 9 minutes to 18 minutes.

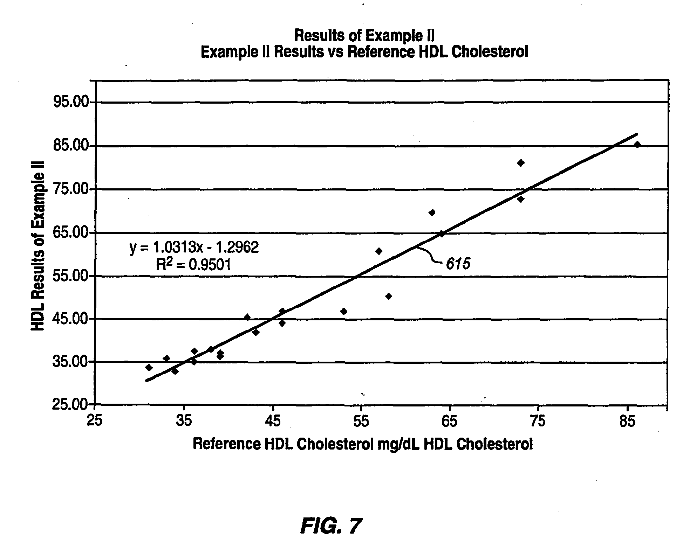

A test strip assembly 50 was made by assembling a sheet of SEFAR^{™} type 76 SK 022, the above two sheets, and a sheet of Blodyne^{™} A material made as discussed in Example I. Circular blanks were cut out and were inserted in a test assembly 20 as shown In FIGS. 1 and 4. In a manner known in the art, a curve similar to that shown In FIG. 6 was then constructed using reflectance measurements from a standard laboratory test for HDL. As also known in the art, the curve was then used to program a Bloscanner 2000 reader available from Polymer Technology Systems, Inc., Indianapolis, IN. The reader was then successfully used to directly read HDL concentrations in milligrams per decider (mg/dl) from a test assembly as described above. FIG. 7 illustrates the results using strips constructed as described in Example II above FIG. 7 is a graph of the HDL cholesterol readings directly read from the reader plotted along the ordinate versus reference HDL cholesterol for the same sample plotted along the abscissa. The line 615 shows where the results would lie if the strip according to the invention gave identical results to the reference test. As can be seen, the plotted points lie very close to the line, and the scatter is essentially random. This is an excellent result since even if the results from two identical reference tests were plotted there would be some scatter. These results show that the dry strip test according to the invention is highly accurate.

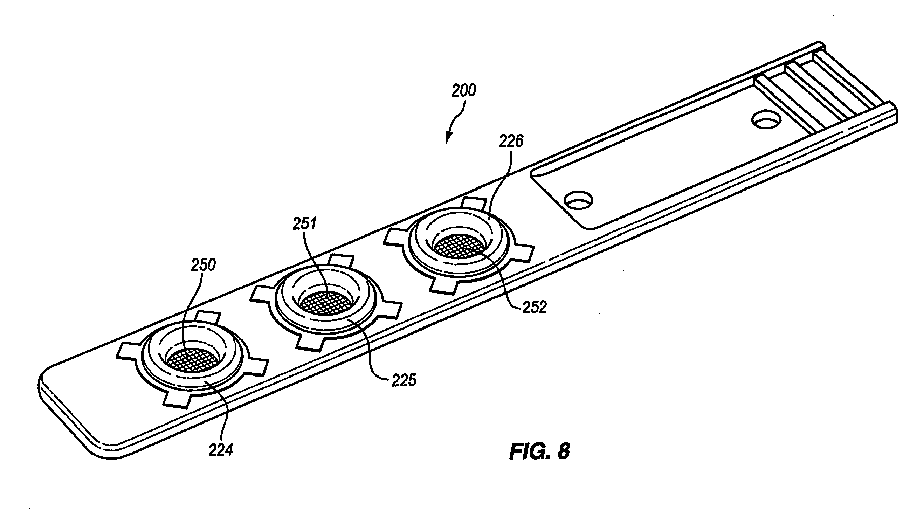

FIG. 8 illustrates another preferred embodiment of a test assembly 200 according to the invention. This embodiment is provided to illustrate that once the design strategy of a non-precipitating dry strip test is disclosed as it has been above, many other non-precipitating dry strip tests can be designed by those skilled in the art. Test assembly 200 is similar to test assembly 20 except there are a plurality of strip holders 224, 225, and 226 each holding a different test strip 260, 261, and 252. Each of the strip holders 224, 225, and 226 have the same structure as strip holder 24. The test strips 250, 251, and 252 preferably have a plurality of layers, and more preferably four layers like test strip 50, though each generally will be impregnated with different chemistry. In particular, the chemicals in the depth filter layer (layer 54 in FIG. 1) will generally be different. In one alternative preferred embodiment, there are two strip holders 224 and 225 each having a different test strip 250 and 251. In this embodiment, test strip 250 contains chemicals for an assay to determine the concentration of HDL + LDL cholesterol, while test strip 251 contains chemicals to determine HDL cholesterol concentration, and the results for test strip 251 are subtracted from the results for test strip 250 to give HDL+LDL-HDL or LDL concentration. From the design strategy disclosure above, one skilled in the art will see that, in this embodiment, an HDL + LDL test strip can be made using an anionic polymer that is larger and has a smaller surface charge density than used for the HDL test described above, and/or surfactants specific to both HDL and LDL. The HDL test strip 251 is the same as the HDL test strip disclosed above.

In another embodiment, test strips 250 and 251 can be used to determine HDL+LDL-HDL = LDL as in the above paragraph, while test strip 252 is used to determine an independent LDL concentration as discussed above. The two LDL concentrations can then be averaged to give a very accurate LDL result because it is determined by two different methods. In this case, the HDL+LDL test strip 250 is as described in the paragraph above, while the HDL test strip 251 and LDL test strip 252 are as disclosed in the LDL chemistry section above.

A feature of the invention is that each layer of the test strip assembly, such as 50 and 450, is engineered to perform specific functions, and at the same time the various layers cooperate so that the test strip assembly as a whole operates to provide more accurate and reliable results. The layers together operate to create a vertical flow of sample liquid essentially across the entire test strip assembly. The red blood cells tend to move slower than the rest of the sample, or get removed from the sample in the layers 54, 56, 454, 456, and therefore, during the time in which the colorimetric reagent is reacting, will be contained in the layers above the reaction layer and will not be in the reaction layer 58, 458. However, the other analytes may or may not be in the reaction layer 58, 458. Since they are rendered non-reactive by the reagents in layer 54, 454, whether or not they are present is not of great importance. Since the non-desired analytes are not precipitated, the pores or channels in the layers 56, 58, 456, 458, and 476 remain open. This allows the sample liquid in the layers 56, 456, 476 adjacent to the reaction layer 58, 458 to participate in the colorimetric reactions. That is, in the embodiment of FIGS. 1 and 4, the majority of the liquid in a layer, such as 56, just above the layer 58, and, in the embodiment of FIG. 5, the majority of the liquid in the layer 458 just above the layer 458 and the majority of the liquid in the layer 476 just below the layer 468, is free to flow into the reaction layer 58, 458 and take part in the colorimetric reaction. This feature creates a larger volume of treated plasma or other bodily liquid. The larger volume directly results in a more accurate measurement. This feature allows the reaction layer 58, 458 to be much thinner than prior art reaction layers and still yield an accuracy associated with reaction layers that are much thicker. 54 and 56 is to contain the red blood cells In this region, and not permit them to get into the reaction layer 58. However, the containment of the red blood cells in layers 54 and 56 does not have to be absolute. Preferably, the containment of the red blood cells is at least 50%, more preferably it is at least 80%, and most preferably it is at least 95%.

In the inventive test, if more bodily fluid than is required for the test is placed In the sample part, such as 45, the fluid in excess of what is required for the test simply fills up the upper portion of the container, such as 80, and does not affect the test. If the excess is too much even for the container, the excess simply overflows the brim 46 and does not affect the test Thus, the bodily fluid analysis system according to the invention is much less sensitive to the amount of bodily fluid supplied than prior art systems.

For this strip holder according to the invention, the percent reflectance versus time curve reaches a minimum and then begins to curve upward. This is because only a well-defined amount of plasma takes part in the reaction, and after that plasma reacts, the color begins to fade as the reactants that produce the color oxidize or otherwise begin to break down, and the slope of the reflectance versus time curve becomes zero. The minimum defines an effective end point that is much easier to measure than a pseudo end point. For example, one can set the electronics to select the effective end point when the value of percent reflectance increases for a predetermined number of measured points, for example three points each taken a second apart. Generally, one will require more than just one increased value of the percent reflectance to determine the effective end point because random noise and other factors can lead to a single increased value for the curve when the curve is actuate still continuing downward. The easier to measure minimum contributes to the increased accuracy of the test strip according to the invention.

A related feature of the invention is that the test strip holder provides a controlled region for vertical flow of the bodily fluid sample. These features, alone and in combination, eliminate or sharply limit leaching or lateral flow of the sample as bodily fluid flows vertically through the layers. This degree of control translates to the ability to obtain accurate test readings from a reduced blood sample. Accurate results can be obtained with a sample size of as low as 4ml and as great as 40ml with the present invention.

Another feature of the invention is that the chemical process is non-precipitating. Precipitation creates particles of precipitate that would tend to clog pores in the test strip 50. Clogging pores impedes the flow of the analyte to the detection membrane, and is not fully predictable, and thus leads to a non-uniform color development. Clogging by precipitates also competes with the filtration of the red blood cells and makes this desirable filtration less effective. A related feature is that the chemistry makes the non-selected bodily fluid components unreactive with the detection compound that creates the colorimetric response. That is, the non-selected bodily fluid components continue to flow in the test strip 50, but are taken out of the detection reaction.

A further feature of the invention is that the test strip assembly, such as 50, preferably does not include any glue, adhesive, or other substance to hold it in place. Such substances can get into the test sample and compromise the test to make it less accurate and reliable.

Another feature of the invention is that the reagents used, particularly those in layer 54, are non-hemolytic. That is, they will not rupture the red blood cells. This prevents the matter from inside the red blood cells from compromising the test. Preferably, the reagents are hypertonic; that is, the reagent in solution has a higher osmotic pressure than the osmotic pressure within the red blood cells. Thus, if there is any flow of water, it will be from within the cell to outside the cell. The reverse could cause the cells to gain water until they rupture. However, the reagents are selected so that the degree of hypertonicity is low. Otherwise, the liquid from with the blood cells could dilute the bodily fluid to be analyzed.

Another feature of the invention is that the interrelationship between reagent formulation and the liquid flow in the materials of the test strip layers is considered. That is, the effect of the reagent on the surface tension of the fluid, and the effect of the resulting surface tension on the rate of flow through the layers are considered. For example, water will generally not flow easily in the layers according to the invention. The membranes, in particular, tend to hold water like a sponge. However, water with the reagents dissolved flows easily in these membranes. This feature helps keep the liquid in the depth filter until the reagents are dissolved.

The test strips, such as 50 and 450, according to the invention are highly sophisticated compared to the prior art test assemblies. The prior art test strips tended to simply include materials, such as fiberglass, that could hold a large amount of bodily fluid and reagent. They succeeded largely because they used large amounts of both bodily fluid and reagent. In contrast, the test strip assemblies according to the invention utilize many different materials that are carefully chosen and engineered, and succeed because they better isolate the desired reaction. Because of this, the test strip assemblies of the invention can operate effectively with a much smaller amount of reagent, and thus are more economical than the prior art test strips.

The design methodology of the invention is a self-consistent and self-reinforcing process. The materials and chemical processes of the invention are carefully engineered so that more accurate and more reliable results can be achieved with a smaller amount of reagent and a correspondingly smaller test strip assembly. Because the results that can be achieved are more accurate and can be achieved with a smaller amount of reagent, more flexibility is permitted in the selection of materials in the layers and the reagents. For example, membranes that retain and hold relatively small amounts of liquid can be selected over fabrics that hold large amounts of fluid, while fabrics that hold large amounts of fluid can also be used advantageously where appropriate. The ability to use a wider variety of materials enables the engineer to design a test that is closely akin to a laboratory analysis. That is, laboratory analyses can be very accurate because the order and timing of the reactions can be carefully controlled. One can add an accurately measured amount of a first reactant to an accurately measured amount of solvent, allow a first reaction to occur, then add an accurately measured amount of second reactant and perform a second reaction, and so on. The ability to use a wide variety of different materials allows one to control the order and timing of the reactions in a similar manner. The first reaction is placed closest to the top in the vertical structure of the test strip assembly. The timing of the second reaction can be controlled by choosing the materials of the first reactant layer and the adjoining layers to control the flow time through the layers, and so on.

While the invention has been disclosed in terms of an HDL or LDL direct assay, it will be evident to those skilled in the art that many aspects of the invention will be useful in other assays. Now that a dry test strip assay has been disclosed that mimics many of the features of a laboratory assay, such as use of a well-defined test volume, reaction order and timing controls using a variety of materials, and the ability to remove red blood cells from the reaction while still providing the above two features, these features may also be used to test for total cholesterol, triglycerides, and many other analytes. Further, now that the advantages of a non-precipitate dry test strip, asymmetric membranes, removal of red blood cells from the detection area without filtering that can clog the system, these features can also be advantageously used for testing of other analytes. Further, although the description has disclosed specific exemplary material layers that perform the features of the invention, now that the functions of the layers and the interrelationships of the layers has been described, many other materials can be substituted which will perform the same functions. In addition, while the invention has been disclosed in terms of specific exemplary reactants, many other reactants that perform the same functions and have some or all of the same advantages can be substituted. Again, while the invention has been disclosed in terms of a particular bodily fluid, i.e., blood and blood plasma, many features of the invention will be useful in testing other bodily fluids, such as urine. Thus, the invention should not be limited to these specific structures, layer materials, reactants, and bodily fluids.

There has been described a novel in vitro, dry test system that is useful to assay for HDL, LDL, and other analytes. It should be understood that the particular embodiments shown in the drawings and described within this specification are for purposes of example and should not be construed to limit the invention, which will be described in the claims below. Further, it is evident that those skilled in the art may now make numerous uses and modifications of the specific embodiments described, without departing from the inventive concepts. For example, other non-precitating chemistries may be used. Equivalent chemicals, membranes or materials may be substituted. The chemicals may be distributed among a lesser or greater number of layers. The layers may be combined, or a plurality of layers may perform the function of one described herein. The non-precipitating chemistry and/or any of its novel features may be used to determine characteristics of other analytes. The non-precipitating chemistry may be used with dry test strips in which electrical parameters, such as resistant, may be altered by the test to indicate the characteristic.

## Claims

1. A method of determining a characteristic selected from one of a plurality of analytes in a bodily fluid, said method comprising:
providing said bodily fluid containing said selected analyte and one or more non-selected analytes;
applying said bodily fluid to a dry test strip; and
reacting said selected one of said analytes with a reactant including 3-{[3-Cholamidopropyl]dimethylammonio}3-propane-sulfonate in said dry test strip to provide an indication of said characteristic while preventing said one or more non-selected analytes from participating in said reaction, without precipitating said one or more non-selected analytes, wherein said selected analyte is low density lipoprotein, wherein said one or more non-selected analytes are prevented from participating by complexing said one or more non-selected analytes and said complexing comprises exposing said bodily fluid to a reagent located on the test strip comprising dextran sulphate, a divalent metal, and wherein said LDL is exposed to polyoxyethylene-polyoxypropylene-polyoxyethylene hybrid as a destabilizing compound.

2. A method as in claim 1 wherein said bodily fluid is blood.

3. A method as in claim 1 wherein said characteristic comprises the concentration of said low density lipoproteins in said blood.

4. A method as in claim 1 wherein said indication is an optical indication.

5. A method as in claim 4 wherein said optical indication is a colorimetric indication.

6. A method as in claim 1, wherein said destabilizing compound has a molecular weight between 2,100 and 6,000.

7. A method as in claim 1, wherein said destabilizing compound has a preponderance of polyoxyethylene.

## Patentansprüche

1. Verfahren zur Bestimmung einer Eigenschaft, ausgewählt aus einer Vielzahl von Analyten in einer Körperflüssigkeit, umfassend:
Bereitstellen der Körperflüssigkeit mit dem ausgewählten Analyten und einem oder mehreren nicht-ausgewählten Analyten;
Aufbringen dieser Körperflüssigkeit auf einen trockenen Teststreifen;
und
Umsetzen dieses einen der besagten Analyten mit einem Reaktanten, beinhaltend 3-{[3-Cholamidopropyl]dimethylammonio}3-propan-sulfonat in diesem trockenen Teststreifen, um eine Indikation auf die Eigenschaft zu liefern, während der eine oder mehrere nicht-ausgewählte Analyten von dieser Reaktion ausgeschlossen werden, ohne dass dieser eine oder mehrere nicht-ausgewählte Analyten ausfallen, wobei dieser ausgewählte Analyt Lipoprotein niedriger Dichte (low density lipoprotein, LDL) ist, wobei dieser eine oder mehrere nicht-ausgewählte Analyten durch Komplexbildung des einen oder mehrerer nicht-ausgewählter Analyten an der Teilnahme gehindert werden, und diese Komplexbildung umfasst die Exposition der Körperflüssigkeit zu einem Reagens, das auf dem Teststreifen umfassend Dextransulfat, ein bivalentes Material, vorhanden ist, und wobei das LDL einem Polyoxyethylen-Polyoxypropylen-Polyoxyethylen-Hybrid als destabilisierende Verbindung exponiert wird.

2. Verfahren nach Anspruch 1, wobei die Körperflüssigkeit Blut ist

3. Verfahren nach Anspruch 1, wobei die Eigenschaft die Konzentration des Lipoproteins niedriger Dichte (low density lipoprotein) in Blut umfasst.

4. Verfahren nach Anspruch 1, wobei die Indikation eine optische Indikation ist.

5. Verfahren nach Anspruch 4, wobei die optische Indikatlon eine colorimetrische Indikation ist.

6. Verfahren nach Anspruch 1, wobei die destabilisierende Verbindung ein Molekulargewicht zwischen 2.100 und 6.000 besitzt.

7. Verfahren nach Anspruch 1, wobei die destabilisierende Verbindung überwiegend aus Polyoxyethylen besteht.

## Revendications

1. Procédé de détermination d'une caractéristique sélectionnée parmi un d'une pluralité d'analytes dans une fluide corporel, ledit procédé consistant à :
fournir ledit fluide corporel contenant ledit analyte sélectionné et un ou plusieurs analytes non sélectionnés ;
appliquer ledit fluide corporel à une bandelette réactive sèche ; et
faire réagir ledit analyte sélectionné parmi lesdits analytes avec un réactif contenant du 3-{[3-cholamidopropyl]diméthylammonio}-3-propane-sulfonate dans ladite bandelette réactive sèche pour fournir une indication de ladite caractéristique tout en empêchant lesdits un ou plusieurs analytes non sélectionnés de participer à ladite réaction, sans précipiter avec lesdits un ou plusieurs analytes non sélectionnés, dans lequel ledit analyte est une lipoprotéine de faible densité, dans lequel on prévient la participation desdits un ou plusieurs analytes sélectionnés en complexant lesdits un ou plusieurs analytes non sélectionnés, ladite complexion comprenant l'exposition dudit fluide corporel à un réactif situé sur la bandelette réactive comprenant du sulfate de dextrane, un métal divalent, et dans lequel ladite LDL est explosée à un hybride de polyoxyéthylène-polyoxypropylène-polyoxyéthylène en tant que composé déstabilisateur.

2. Procédé selon la revendication 1, dans lequel ledit fluide corporel est du sang.

3. Procédé selon la revendication 1, dans lequel ladite caractéristique comprend la concentration desdites lipoprotéines de faible densité dans ledit sang.

4. Procédé selon la revendication 1, dans lequel ladite indication est une indication optique.

5. Procédé selon la revendication 4, dans lequel ladite indication optique est une indication colorimétrique.

6. Procédé selon la revendication 1, dans lequel ledit composé déstabilisateur a une masse moléculaire entre 2100 et 6000.

7. Procédé selon la revendication 1, dans lequel ledit composé déstabilisateur présente une prépondérance de polyoxyéthylène.
